# EUROPEAN PATENT APPLICATION

(11) **EP 1 489 412 A1**
(43) Date of publication of application: **22.12.2004**
(21) Application number: 04252111.2
(22) Date of filing: 08.04.2004
(51) Int. Cl.: G01N 33/00, B01D 53/86, B01D 15/08

(54) **Method and apparatus for measuring mercury contained in gaseous medium**

(30) Priority: 27.05.2003 JP 2003148808
(71) Applicant: Central Research Institute of Electric Power Industry, Tokyo 100-8126 (JP); Nippon Instruments Corporation, Osaka 569-1146 (JP)
(72) Inventor: Makino, Hisao, Yokosuka-shi Kanagawa 240-0196 (JP); Shirai, Hiromi, Yokosuka-shi Kanagawa 240-0196 (JP)
(74) Representative: Skone James, Robert Edmund

(57) **Abstract**

To provide a method of and an apparatus for continuously measuring elemental mercury and bivalent mercury both contained in a gaseous medium fractionally with a simplified structure, the concentration of a total mercury (Metallic Mercury + Bivalent Mercury) and the concentration of elemental mercury contained in gases are measured continuously and fractionally. In the practice of this mercury measuring method, a first column 1, filled with a first fixed catalyst, and a second column 11, filled with a second fixed catalyst, are fluid connected in parallel relation to each other. The gases G are introduced into those first and second columns 1 and 11. In the first column 1, the first fixed catalyst collects and removes the bivalent mercury, but passes only the elemental mercury in the gases through the first column. In the second column 11, the second fixed catalyst reduces the bivalent mercury into elemental mercury and passes through the second column 11 the elemental mercury in the gases containing the elemental mercury into which the bivalent mercury has been reduced. The concentration of the elemental mercury in the gases, from which the bivalent mercury has been removed after passage thereof through the first column 1 and, also, the concentration of the elemental mercury in the gases into which the bivalent mercury has been reduced after passage thereof through the second column 11 are measured as the concentration of the elemental mercury contained in sampled gases and as the concentration of the total mercury in the sampled gases, respectively by utilization of first and second mercury measuring instruments.

## Description

The present invention generally relates to a method of and an apparatus for measuring the concentration of mercury contained in a gaseous medium such as, for example, a flue gas emitted from an incineration facility utilizing a fossil fuel, an industrial waste incineration facility or a chemical plant performing a chemical process and, more particularly, to such method and such apparatus for use in monitoring the concentration of the mercury content in the gaseous medium according to the chemical conformation.

It is well known that the flue gas emitted from chemical plants such as an incineration facility utilizing a fossil fuel, an industrial waste incineration facility or the like contains mercury principally in two chemical conformations, that is, bivalent mercury (Hg²⁺) and elemental mercury (Hg⁰) of inorganic mercury. Of them, the elemental mercury is hardly insoluble in water and is susceptible to dispersion to the atmosphere, tending to constitute one of the causes of atmospheric contamination. On the other hand, the bivalent mercury is soluble in water, tending to constitute one of causes of water and soil contamination. As such, since the elemental mercury and the bivalent mercury bring about differing impacts on the environment, the need has been recognized to measure those components of mercury individually and fractionally.

For measurement of mercury contained in flue gases, a total mercury (inorganic mercury and organic mercury) measurement method as an on-line analysis (a flow method), a measuring method utilizing a gold amalgam as a batch method (such as stipulated in JIS K 0222) and the like have long been available in the art. Also, as a batch method, the Japanese Laid-open Patent Publication No. 2001-221787, filed by the same applicant as that of the present invention, discloses a method of measuring the concentration of mercury contained in naphtha and LPG (liquefied petroleum gas).

However, it has been found that the batch method requires not only a long time to complete a single measurement, but also collection of the flue gas each time the measurement is carried out. Accordingly, the batch method currently available in the art is felt insufficient in real-time determination of the concentration of mercury contained in flue gases emitted from an incineration facility utilizing a fossil fuel, an industrial waste incineration facility or the like and, as such, a quick countermeasure can hardly be taken to minimize the obnoxious emission. On the other hand, with the prior art flow method, the flue gases need be brought into contact with an aqueous solution of, for example, potassium permanganate and, accordingly, resulting in such a problem that the measuring apparatus tends to be bulky and complicated in structure.

In view of the foregoing, the present invention has for its object to provide a method of continuously measuring elemental mercury and bivalent mercury both contained in a gaseous medium fractionally.

Another important object of the present invention is to provide a measuring apparatus of a simplified structure that can be used in the practice of the measuring method of the kind referred to above.

In order to accomplish these objects of the present invention, the present invention in accordance with one aspect thereof provides a method of measuring continuously and fractionally the concentration of a total mercury (Metallic Mercury + Bivalent Mercury) and the concentration of elemental mercury contained in gases. In the practice of this mercury measuring method, a first column, filled with a first fixed catalyst, and a second column, filled with a second fixed catalyst, are fluid connected in parallel relation to each other, and the gases are introduced into those first and second columns. In the first column, the first fixed catalyst collects and removes the bivalent mercury, but passes only the elemental mercury in the gases through the first column. In the second column, the second fixed catalyst reduces the bivalent mercury into elemental mercury and passes through the second column the elemental mercury in the gases containing the elemental mercury into which the bivalent mercury has been reduced. The concentration of the elemental mercury in the gases, from which the bivalent mercury has been removed after passage thereof through the first column and, also, the concentration of the elemental mercury in the gases into which the bivalent mercury has been reduced after passage thereof through the second column are measured as the concentration of the elemental mercury contained in sampled gases and as the concentration of the total mercury in the sampled gases, respectively by the utilization of first and second mercury measuring instruments.

According to the present invention, when the gases are introduced into the first column, the bivalent mercury contained in the gases can be collected and removed by the first fixed catalyst contained in the first column, allowing only the elemental mercury to pass therethrough for introduction into one of the mercury measuring instruments which subsequently measures the concentration of the elemental mercury contained in the gases. On the other hand, when the gases are introduced into the second column, the bivalent mercury contained in the gases can be reduced into the elemental mercury by the second fixed catalyst contained in the second column, and the total mercury including the reduced elemental mercury and the elemental mercury originally contained in the gases is subsequently introduced into the other of the mercury measuring instruments which subsequently measures the concentration of the total mercury in the gases. The concentration of the bivalent mercury contained in the gases can be calculated by subtracting the concentration of the elemental mercury from the concentration of the total mercury. Accordingly, in the practice of the present invention, the gases need not be brought into contact with an aqueous solution such as required in the practice of the conventional flow method and, therefore, with a simplified structure, the respective concentrations of the elemental mercury and the bivalent mercury both contained in the gases can be continuously measured fractionally and, yet, this measurement can be easily accomplished in a short length of time as compared with that required in the practice of the conventional batch method.

In a preferred embodiment of the present invention, a component likely to interfere with the mercury measurement which includes a sulfurous acid gas, may be removed from the gases after the gases have been passed through each of the first and second columns. By so doing, since the gases to be measured contains no interfering component, an accurate measurement of the elemental mercury and the bivalent mercury can advantageously be accomplished.

In another preferred embodiment of the present invention, sampling of the gases, measurement of the concentration of mercury contained in the gases by the use of said mercury measuring method and display of measurements of the mercury are carried out continuously and on real time basis.

According to this feature, since the respective concentrations of the elemental mercury and the bivalent mercury contained in the gases can be determined on real time basis with no need to use of any liquid absorbent, the cycle of servicing can be prolonged and the measurement can be performed for an extended period of time and, accordingly, when the present invention is applied to a chemical plant such as, for example, an incineration facility utilizing a fossil fuel or an industrial waste incineration facility, a countermeasure to minimize the obnoxious emission can be taken quickly.

The present invention in accordance with another aspect thereof also provides an apparatus for measuring continuously and fractionally the concentration of a total mercury (Metallic Mercury + Bivalent Mercury) and the concentration of elemental mercury contained in gases, which apparatus executes the mercury measuring method discussed above.

In any event, the present invention will become more clearly understood from the following description of a preferred embodiment thereof, when taken in conjunction with the accompanying drawings. However, the embodiment and the drawing are given only for the purpose of illustration and explanation, and are not to be taken as limiting the scope of the present invention in any way whatsoever, which scope is to be determined by the appended claims. In the accompanying drawings, like reference numerals are used to denote like parts throughout the several views, and:

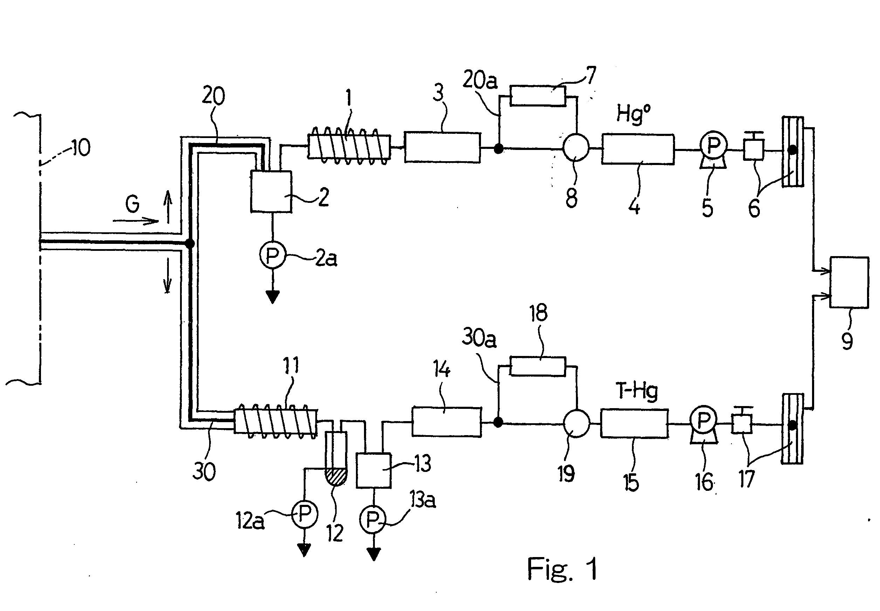

Fig. 1 is a schematic diagram showing an apparatus for measuring the concentration of mercury contained in a gaseous medium according to a preferred embodiment of the present invention.

Hereinafter, a preferred embodiment of the present invention will be described with particular reference to Fig. 1 which illustrates a schematic diagram showing an apparatus for measuring the concentration of mercury contained in a gaseous medium. The measuring apparatus shown therein includes first and second gas intake ducts 20 and 30 fitted to, for example, a side wall of an exhaust flue (pipe) 10 in, for example, a chemical plant for discharge of flue gases and fluid connected parallel to the exhaust flue 10 and also to each other. The first gas intake duct 20 is in turn fluid connected with a dehumidifier 2 having a drain pump 2a and then with a first column 1 filled with a first fixed catalyst capable of collecting and removing bivalent mercury (Hg²⁺) contained in the gases G, but allowing passage therethrough of only elemental mercury (Hg⁰) contained in the same gases G. The first column 1 is in turn fluid connected with a first interfering component removal column 3 and then with a first mercury measuring instrument 4 for measuring the concentration of Hg⁰ that has passed through the first column 1. The gases G within the exhaust flue 10 can be pumped into the first gas intake duct 20 by a first air pump 5 which is fluid connected with and positioned downstream of the first mercury measuring instrument 4. The flow of the gases G induced by the first air pump 5 can be controlled by a first controller 6 that is connected with and positioned downstream of the first air pump 5. The first fixed catalyst filled in the first column 1 may be suitably employed in the form of, for example, activated alumina (Al₂O₃) which is known to be excellent in collecting Hg²⁺ without collecting Hg⁰.

Similarly, the second gas intake duct 30 fluid connected parallel to the first gas intake duct 20 is in turn fluid connected with a second column 11 filled with a first fixed catalyst capable of reducing Hg²⁺ in the gases G, into Hg⁰ and allowing passage therethrough of the reduced Hg⁰ and Hg⁰ originally contained in the gases G and, then, with a gas-liquid separator 12 having a drain pump 12a. The gas-liquid separator 12 is in turn fluid connected with a second interfering component removal column 14 through a dehumidifier 13 having a drain pump 13a. The second interfering component removal column 14 is then fluid connected with a second mercury measuring instrument 15 for measuring the concentration of the total elemental mercury (Metallic Mercury + Bivalent Mercury, T-Hg), which corresponds to the sum of Hg⁰, which has been reduced by the second column 11, and Hg⁰ originally contained in the gases G, that is, for measuring the concentration of the total elemental mercury (T-Hg)as converted into the concentration of the summed Hg⁰. The gases G within the exhaust flue 10 can be pumped into the second gas intake duct 30 by a second air pump 16 which is fluid connected with and positioned downstream of the second mercury measuring instrument 15. The flow of the gases G induced by the second air pump 16 can be controlled by a second controller 17 that is connected with and positioned downstream of the second air pump 16. The second fixed catalyst filled in the second column 11 may be suitably employed in the form of, for example, tin chloride (SnCl₂) which is known to be excellent in reducing Hg²⁺.

For each of the first and second mercury measuring instruments 4 and 15, any known mercury measuring instrument, for example, a flameless atomic absorption photometer can be employed. Also, the first and second controllers 6 and 17 have a respective output end that is electrically connected with a corresponding monitor display 9 so that measurements performed by the respective mercury measuring instrument 4 and 15 can be displayed on real time basis.

In the embodiment so far shown in Fig. 1, a portion of the first gas intake duct 20 between the first interfering component removal column 3 and the first mercury measuring instrument 4 is fluid connected with a first gas filter 7 through a branch passage 20a which is in turn fluid connected with a first electromagnetic three-way switching valve 8 positioned immediately upstream of the first mercury measuring instrument 4. A portion of the gases G flowing through the gas filter 7 and the gases G flowing directly through the first gas intake duct 20 can be selectively supplied into the first mercury measuring instrument 4 one at a time by the first electromagnetic three-way switching valve 8 which performs a regular flow switching.

Similarly, a portion of the second gas intake duct 30 between the second interfering component removal column 14 and the second mercury measuring instrument 15 is fluid connected with a second gas filter 18 through a branch passage 30a which is in turn fluid connected with a second electromagnetic three-way switching valve 19 positioned immediately upstream of the second mercury measuring instrument 15. A portion of the gases G flowing through the second gas filter 18 and the gases G flowing directly through the second gas intake duct 30 can be selectively supplied into the second mercury measuring instrument 15 one at a time by the second electromagnetic three-way switching valve 19 which performs a regular flow switching.

Each of the gas filters 7 and 18 referred to above is operable to remove Hg⁰ contained in the gases G to provide the gases containing no mercury. At the timing the gases with no mercury flow, a zero base correction takes place in each of the first and second mercury measuring instruments 4 and 15. The sequences of operation of the measuring apparatus are controlled by a control apparatus not shown in the drawing.

The measurement of the mercury concentration performed by the measuring apparatus of the structure described above will now be described. As a matter of design, the following sequence takes place under the control performed by the control device.

At the outset, when the first and second air pumps 5 and 16 are driven under the controls performed by the first and second controllers 6 and 17, respectively, a predetermined quantity of the gases G are introduced from the exhaust flue 10 into the first and second gas intake ducts 20 and 30 and are then sampled. The gases G introduced into the first gas intake duct 20 are introduced into the first column 1 then controlled to a predetermined temperature. As the gases G flows through the first column 1, Hg²⁺ contained in the gases G is collected and removed by the first fixed catalyst, with the gases G subsequently emerging outwardly from the first column 1 without the gaseous Hg⁰ having been collected by the first fixed catalyst. Subsequently, the gases G are supplied to the dehumidifier 2 at which the moisture content of the gases G is cooled and dehumidified, with the resultant condensed water subsequently discharged to the outside by the drain pump 2a.

Thereafter, a component likely to interfere with the mercury measurement which includes, for example, a sulfurous acid gas is removed by the first interfering component removal column 3. Also, the gases G from which the interfering component has been removed is supplied to the first mercury measuring instrument 4 after having passed through or without passing through the first gas filter 7 depending on the switching position of the first electromagnetic three-way switching valve 8. With the gases G so supplied to the first mercury measuring instrument 4, the concentration of Hg⁰ contained in the gases G with Hg²⁺ having been removed is continuously measured by the first mercury measuring instrument 4, the measurement of which is subsequently displayed by the monitor display 9. The first column 1 referred to above is regenerated by heating it prior to breakthrough by Hg²⁺.

On the other hand, the gases G introduced into the second gas intake duct 30 flows into the second column 11 at which Hg²⁺ contained in the gases G is reduced into Hg⁰ by the second fixed catalyst contained therein. Subsequently, the gases G containing the reduced Hg⁰ and Hg⁰ originally contained therein are supplied to the gas-liquid separator 12 at which Hg²⁺ dissolved in a small quantity in the drain is vaporized to facilitate reduction, followed by return thereof to the second gas intake duct 30. The drain overflowing is discharged to the outside by the action of the drain pump 12a.

Thereafter, the gases G are supplied to the second dehumidifier 13 at which the moisture content of the gases G is cooled and dehumidified, with the resultant condensed water subsequently discharged to the outside by the drain pump 13a. Also, the dehumidified gases G is subsequently supplied to the second interfering component removal column 14 at which an interfering component such as, for example, a sulfurous acid gas is removed. Also, the gases G from which the interfering component has been removed is supplied to the second mercury measuring instrument 15 after having passed through or without passing through the second gas filter 18 depending on the switching position of the second electromagnetic three-way switching valve 19. With the gases G so supplied to the second mercury measuring instrument 15, the concentration of the total mercury (T-Hg), which corresponds to the sum of the reduced Hg⁰ contained in the gases G, and Hg⁰ originally contained in the gases G, is continuously measured by the second mercury measuring instrument 15, the measurement of which is subsequently displayed by the monitor display 9 together with the measurement given by the first mercury measuring instrument 4. Specifically, by subtracting the concentration of Hg⁰ from the concentration of the total mercury (Metallic Mercury + Bivalent Mercury), the concentration of Hg²⁺ contained in the gases G is automatically calculated and displayed by the monitor display 9.

Thus, in the practice of the present invention, the gases need not be brought into contact with an aqueous solution such as required in the practice of the conventional flow method and, therefore, with a simplified structure, the respective concentrations of Hg⁰ and Hg²⁺ both contained in the gases can be continuously measured fractionally and, yet, this measurement can be easily accomplished in a short length of time as compared with that required in the practice of the conventional batch method. Also, since after the gases have passed through the first and second columns 1 and 11, the interfering component such as sulfurous acid gas contained in the gases can be removed by the first and second interfering component removal columns 3 and 14, an accurate measurement of the mercury concentration can advantageously be accomplished.

Again, by the drive of the first and second air pumps 5 and 16 the gases G within the exhaust flue 10 are sampled and are subsequently introduced into the first and second gas intake ducts 20 and 30, respectively, and as the gases G flow through the first and second columns 1 and 11, disposed on the first and second gas intake ducts 20 and 30, various processes are applied to the mercury contained in different chemical conformation in the gases, followed by the mercury measurement performed by the first and second mercury measuring instruments 4 and 15, respectively, with the measurements subsequently displayed by the monitor display 9. Accordingly, by this sequence, determination of the mercury concentration in the gases G can be advantageously accomplished on real time basis. Because of this, when the mercury measuring apparatus of the present invention is applied to such a chemical plant as, for example, an incineration facility utilizing a fossil fuel or an industrial waste incineration facility, it is advantageously possible to take a quick countermeasure to minimize the obnoxious emission.

## Claims

1. A method of measuring continuously and fractionally the concentration of a total mercury (Metallic Mercury + Bivalent Mercury) and the concentration of elemental mercury contained in gases, said method comprising:
fluid connecting a first column, filled with a first fixed catalyst, and a second column, filled with a second fixed catalyst, in parallel relation to each other;
introducing the gases into the first and second columns;
causing the first fixed catalyst in the first column to collect and remove the bivalent mercury and passing only the elemental mercury in the gases through the first column;
causing the second fixed catalyst in the second column to reduce the bivalent mercury into elemental mercury and passing through the second column the elemental mercury in the gases containing the elemental mercury into which the bivalent mercury has been reduced; and
by utilization of first and second mercury measuring instruments, measuring the concentration of the elemental mercury in the gases, from which the bivalent mercury has been removed after passage thereof through the first column, as the concentration of the elemental mercury contained in sampled gases and measuring the concentration of the elemental mercury in the gases into which the bivalent mercury has been reduced after passage thereof through the second column, as the concentration of the total mercury in the sampled gases, respectively.

2. The mercury measuring method as claimed in Claim 1, further comprising removing a component likely to interfere with the mercury measurement which includes a sulfurous acid gas, from the gases after the gases have been passed through each of the first and second columns.

3. A method of measuring the concentration of mercury contained in gases, wherein sampling of the gases, measurement of the concentration of mercury contained in the gases by the use of the mercury measuring method as claimed in Claim 1 and display of measurements of the mercury are carried out continuously and on real time basis.

4. An apparatus for measuring continuously and fractionally the concentration of a total mercury (Metallic Mercury + Bivalent Mercury) and the concentration of elemental mercury contained in gases, said apparatus comprising:
a first column filled with a first fixed catalyst for collecting and removing the bivalent mercury, contained in gases introduced thereinto, but allowing only the elemental mercury in the gases to pass therethrough;
a second column fluid connected parallel to the first column and filled with a second fixed catalyst for reducing the bivalent mercury, contained in gases introduced thereinto, into elemental mercury and passing therethrough the elemental mercury in the gases containing the elemental mercury into which the bivalent mercury has been reduced; and
first and second mercury measuring instruments for measuring the concentration of the elemental mercury in the gases, from which the bivalent mercury has been removed after passage thereof through the first column, as the concentration of the elemental mercury contained in sampled gases and measuring the concentration of the elemental mercury in the gases into which the bivalent mercury has been reduced after passage thereof through the second column, as the concentration of the total mercury in the sampled gases, respectively.

5. The mercury measuring apparatus as claimed in Claim 4, further comprising an interfering component removal column positioned downstream of each of the first and second columns with respect to a direction of supply of the gases, said interfering component removal column being operable to remove a component likely to interfere with the mercury measurement which includes a sulfurous acid gas, from the gases.
